# EUROPÄISCHE PATENTSCHRIFT

(11) **EP 2 852 438 B1**
(45) Veröffentlichungstag und Bekanntmachung des Hinweises auf die Patenterteilung: **29.06.2016**
(21) Anmeldenummer: 13724273.1
(22) Anmeldetag: 22.05.2013
(51) Int. Cl.: A61P 25/20, A61P 43/00, A61K 31/7004, A61K 31/711, A61K 45/06, A61K 31/122, A61K 31/194, A61K 31/197, A61K 31/375, A61K 31/405, A61K 31/455, A61K 31/525, A61K 31/7084, A61K 31/714

(54) **ZUSAMMENSETZUNG ZUR BEHANDLUNG EINER STÖRUNG DES CIRCADIANEN RHYTHMUS**
COMPOSITION FOR TREATING A CIRCADIAN RHYTHM DISORDER
COMPOSITION DESTINÉE AU TRAITEMENT D'UN TROUBLE DU RYTHME CIRCADIEN

(30) Priorität: 23.05.2012 DE 102012104451
(43) Veröffentlichungstag der Anmeldung: 01.04.2015
(73) Patentinhaber: Ruhlmann, Jürgen, 53125 Bonn (DE)
(72) Erfinder: RUHLMANN, Benjamin Jürgen, 53125 Bonn (DE); RUHLMANN, Jürgen, 53125 Bonn (DE)
(74) Vertreter: Moser Götze & Partner Patentanwälte mbB
(86) Internationale Anmeldenummer: PCT/EP2013/060541
(87) Internationale Veröffentlichungsnummer: WO 2013/174882

(56) Entgegenhaltungen:
- WO-A1-95/17199
- US-A1- 2003 021 772

## Beschreibung

Die vorliegende Erfindung betrifft eine Komposition umfassend NADH und D-Galaktose zur Behandlung einer Störung des circadianen Rhythmus und betrifft insbesondere die Verwendung einer bestimmten Kombination von Vitaminen, Koenzymen, Mineralien und Galaktose zur Erleichterung der Synchronisation eines zirkadianen Rhythmus, insbesondere des Jetlags.

Im Zusammenhang mit der Störung von circadianen Rhythmen bestehen Zeitverschiebungen durch Langstreckenflüge, Schichtarbeiten, Arbeiten unter extremen Bedingungen mit künstlichen Schlaf-Wach-Rhythmen im Vordergrund.

Es ist bekannt, Melatonin als Medikament zur schnelleren Anpassung an Zeitverschiebungen, z. B. nach Langstreckenflügen, zu verabreichen. Melatonin ist ein Tryptophan-Derivat, es wird von der sogenannten Pinealdrüse produziert. In den Pinealocyten, den Zellen dieser Drüse, wird Tryptophan aus dem Blut über mehrere Biosyntheseschritte zu Melatonin umgewandelt. Melatonin ist in den USA als Nahrungsergänzungsmittel, jedoch nicht als Arzneimittel zugelassen. In der Bundesrepublik Deutschland sind Melatonin-Präparate auf dem freien Markt nicht erhältlich, lediglich in Form des Arzneimittels Circadin. Die Kombination der oben genannten Substanzen stimuliert u. a. die Bildung des Melatonins. Soweit bislang heute bekannt ist, hat Melatonin die oben beschriebene Wirkung aufgrund folgender mechanistischer Erklärungsversuche: Das im Blutkreislauf zirkulierende Melatonin wirkt hauptsächlich an zentralen Bindungsstellen in bestimmten Arealen des Hypothalamus und der Adenohypophyse. Soweit bekannt ist, zeigen die Melatoninkonzentrationen beim Menschen im Serum ein circadianes Muster mit hohen Konzentrationen in der Nacht und niedrigen während des Tages. Dieses Muster wird durch die symmetrische Innervation des Pinealorgans erzeugt. Der tägliche Licht-Dunkel-Wechsel wird bei Säugetieren über die Retina wahrgenommen. Diese Information wird anschlie0end neuronal über den retinohypothalamischen Trakt zum Nucleus suprachiasmaticus (SCH), der sogenannten circadianen Uhr, weitergeleitet. Nach weiteren Schaltstellen erreichen von dort postganglionäre sympathische Fasern, die ihren Ursprung im Ganglion cervicale superior (GCS) haben, das Pinealorgan. Während der Dunkelphase werden diese Fasern durch die Aktivität des SCH stimuliert, was zu einer Freisetzung des Neurotransmitters Noradrenalin an den im Pinealorgan liegenden Nervenendigungen führt. Noradrenalin interagiert mit vorwiegend ß-adrenergen Rezeptoren auf der Membran von Pinealocyten und regt über das cAMP second-messender-System die Aktivität des N-Acetyltransferase an. Diese Reaktion stellt nach derzeitigen Erkenntnissen den geschwindigkeitsbestimmenden Schritt der Melatoninsynthese dar.

Auf das oben beschriebene Melatoninsystem wirkt Licht als Zeitgewebe, der den circadianen Rhythmus synchronisiert. Bei Lichtexposition kommt es zu einer Einstellung der Noradrenalin-Ausschüttung. Daraus resultiert eine Hemmung der Melatoninsynthese und so folgt ein Abfall der Konzentration des Melatonins im Blut. Beim Menschen hat Melatonin im Serum eine Halbwertszeit von etwa 35 bis 50 Minuten. Melatonin wirkt somit als endokrines Signal für die Länge der Nacht, denn die Dauer der nächtlichen Melatoninabgabe ist proportional zur Länge der Dunkelphase. Der Organismus ist so in der Lage, anhand des Melatoninprofils die jeweilige Tages- und Jahreszeit, in der er sich befindet, zu bestimmen. Beim Menschen, der weniger ausgeprägte saisonale Anpassungen zeigt als Tiere, spielt Melatonin vor allem eine Rolle bei der Regulation des Schlaf-Wach-Zyklus, weshalb Melatonin zur Erleichterung der Anpassung nach Langstreckenflügen, also beim sogenannten Jetlag, erfolgreich eingesetzt wird (ARENDT J., ALDHOUS M., MARKS M. (1987): Some effects of jet-lang und their treatment by melatonin; Ergonomics 30: 1393 - 1397 (1987); ARENDT J., ALDHOUS M., MARKS V. (1986): Alleviation of jet-lag by melatonin: preliminary results of controlled double-blind trial, BMJ 292: 1170.

Aus der US 2003/0021772 A1 ist es bekannt, NADH alleine zur Linderung von Störungen beruhend auf Schlafentzug oder Jetlag einzusetzen.

Aufgabe der vorliegenden Erfindung ist es, eine mögliche Alternative zu Melatonin bzw. NADH alleine als Mittel zur Anpassung an einen externen Zeitgeber zur Verfügung zu stellen.

Diese Aufgabe wird durch die in Anspruch 1 wiedergegebene Komposition gelöst.

Erfindungsgemäß ist erkannt worden, dass eine Komposition umfassend NADH und D-Galaktose zur Behandlung einer Störung des zirkadianen Rhythmus geeignet ist und zur Erleichterung der Synchronisation des zirkadianen Rhythmus durch einen externen Zeitgeber, wie bei Licht-Dunkel-Wechsel, Tag-Nacht-Rhythmus-Änderungen, Zeitverschiebungen, wie sie bei Langstreckenflügen und dem sog. Jetlag auftreten, geeignet ist.

Die Verwendung der o. g. Kombination ist jedoch auch geeignet, um beispielsweise bei erzwungenen Änderungen des Aktivitätsrhythmus bei Schichtarbeitern eingesetzt zu werden.

NADH ist ein Koenzym und die energiereiche, reduzierte Form von NAD+. Es dient im oxidativen Stoffwechsel als energielieferndes Koenzym der Atmungskette, wobei ATP generiert wird. Bei der Oxidation des NADH gibt dieses Elektronen ab und überträgt sie so im Rahmen der intrazellulären Knallgasreaktion auf Sauerstoff, dabei entstehen NAD+ und Wasser. NAD+ ist u. a. auch ein Koenzym von beispielsweise der Alkoholhydrogenase, die Alkohol oxidiert. NAD+ wird im Körper einerseits aus Niacin (Vitamin B3, Nikotinsäure) oder Nikotinamid, andererseits auch aus den Abbauprodukten der Aminosäure Tryptophan produziert.

Diese beiden Ausgangsstoffe sind essentiell, so dass Mangelerscheinungen von NAD+ möglich sind, was aber durch die Redundanz beider Stoffwechselwege selten ist.

Aufgrund der heute eher reduzierten Zufuhr von Niacin und Tryptophan erscheinen niedrig normale NAD+-Spiegel häufiger.

NADH ist eines der stärksten Antioxidationsmittel, die Wirkung liegt einerseits darin, die Oxidationsprozesse im Körper, also Abbauprozesse durch die Reaktion mit Sauerstoff, aufzuhalten oder zu verlangsamen und andererseits darin, der Zelle aggressive oder belastende Substanzen aus der Umwelt abzufangen.
Vor allem bei der Energiegewinnung spielt NADH eine entscheidende Rolle, es ist der wichtigste Elektronentransporteur innerhalb der energieproduzierenden Prozesse. Ohne die Wirkung von NADH kann der Mensch seine Energie nicht optimal mobilisieren. Er fühlt sich energielos, müde und abgeschlagen.
Aufgrund unserer heutigen Lebensweise/Ernährung ist die Versorgung mit NADH nicht mehr ausreichend gesichert, dabei geht z. B. durch das Kochen von Fleisch und Gemüse schon ein Großteil der NADH-Wirkung verloren.

Je mehr NADH der Zelle zur Verfügung steht, umso mehr Energie kann die Zelle produzieren.

Durch NADH wird die Herstellung der körpereigenen "Glückshormone" Dopamin und Noradrenalin angeregt bzw. beschleunigt. Dies wirkt sich maßgeblich auf die Konzentrations- und individuelle Leistungsfähigkeit aus, auch auf die allgemeine Grundstimmung, die Motivation und das individuelle Energiepotential, die Verbesserung jeglicher Leistungsfähigkeit wird verbessert. NADH reduziert den Jetlag, kann Schlafmangel kompensieren und auch die Libido steigern. Da NADH freie Radikale höchst effektiv bekämpft, werden auch die Körperzellen nachhaltig geschützt (Literatur Prof. Dr. J. Birkmayer: NADH (Koenzym 1), biologische Funktion und therapeutische Anwendungen, DVD-Wissen.com).

Galaktose ist ein natürlich vorkommender Einfachzucker. Die Galaktose kann im Gegensatz zur Glukose insulinunabhängig über ein Konzentrationsgefälle in die Zelle, vor allem die Hirnzelle gelangen. Dort wird Galaktose schnell vollständig in Glukose umgewandelt, wodurch die Hirnfunktion verbessert wird.

Insbesondere hat sich herausgestellt, dass die Komposition besonders wirksam ist, wenn sie ferner mindestens eine oder mehrere Substanzen aus der Gruppe bestehend aus L-Tryptophan, Nikotinsäure oder Nikotinamid (Vitamin B3), Vitamin B2, Vitamin B12, Magnesium, L-Carnitin, Koenzym Q10, Vitamin C oder Vorläufer der zuvor genannten umfasst.

Unter Vorläufer werden insbesondere "Vorstufen" der genannten Stoffe verstanden, die vom Körper in die genannten Stoffe umgesetzt werden.

Tryptophan ist eine aromatische Aminosäure und Bestandteil von Proteinen und Peptiden. Es kann im menschlichen Organismus nicht hergestellt werden, dieser ist auf die Zufuhr mit der Nahrung angewiesen. L-Tryptophan ist der Vorläufer des Serotonins. Stress - und der hierdurch bedingt erhöhte Cortisol-Spiegel - führt zu einer Aktivierung des Tryptophan abbauenden Enzyms Tryptophan-Pyrrolase. Eine Überdosierung von L-Tryptophan ist nur schwer möglich, da L-Tryptophan selbst der Hauptaktivator seines abbauenden Enzyms Tryptophan-Pyrrolase ist.

Niacin oder auch Nikotinsäure genannt ist ein Vitamin aus dem B-Komplex. Es findet sich in allen lebenden Zellen und bildet einen wichtigen Baustein verschiedener Coenzyme, in dieser Form ist es von zentraler Bedeutung für den Stoffwechsel von Eiweißen, Fetten und Kohlehydraten. Nikotinsäure hat eine antioxidative Wirkung und ist u. a. auch wichtig für die Regeneration von Hautmuskeln, Nerven und DNA. Der durchschnittliche Tagesbedarf beträgt für Frauen 13 bis 15 mg, für Männer 15 bis 20 mg.

Koenzym Q10, oder auch Ubichinon 10 wird zum Teil über die Nahrung aufgenommen, aber auch im Körper selbst produziert. Es ist als Koenzym an der oxidativen Phosphorylierung beteiligt, über die 95 % der gesamten Körperenergie (ATP) im Rahmen der "kontrollierten Knallgasreaktion" erzeugt wird. Die Elektronen zur Reduktion des Ubichinons entstammen der Oxidation des NADH.

Vitamin B-Komplex: In dieser Vitamingruppe werden acht Vitamine zusammengefasst, die alle als Vorstufen für Coenzyme dienen. Es sind B1-Thiamin, B2, Rhiboflavin, B6 Pyridoxin, B12 Cobalamin, B7 Biotin, B9 Folsäure, B3 Nikotinsäure und B5 Pantothensäure.

Vitamin C ist ein Radikalenfänger und hat eine antioxidative Wirkung, es stellt ein wichtiges Coenzym für ein Enzym bei der Biosynthese des Proteins Kollagen dar. Außerdem wandelt es Prolinreste in Hydroxyprolin, das für den stabilen Kollagenaufbau unerlässlich ist. Vitamin C ist bei der Hydroxylierung von Stereoiden ein wichtiger Kofaktor, außerdem spielt es eine wichtige Rolle beim Aufbau von Aminosäuren, wie beispielsweise dem L-Thyrosin. Zudem ist es bei der Umwandlung von Dopamin zu Noradrenalin, im Cholesterinstoffwechsel und bei der Carnitinbiosynthese notwendig. Mit Niacin und Vitamin B6 steuert Vitamin C die Produktion von L-Carnitin, das für die Fettverbrennung in der Muskulatur benötigt wird. Zudem begünstigt es die Eisenresorption im Dünndarm.

Magnesium ist für alle Organismen unentbehrlich. Es ist in etwa 300 Enzymreaktionen als Enzymbestandteil oder Koenzym beteiligt. Magnesiumionen fungieren als second Messenger im Immunsystem. Magnesiummangel löst beim Menschen u. a. auch Kopfschmerzen, Konzentrationsmangel, Müdigkeit, allgemeines Schwächegefühl, Herzrhythmusstörungen und Muskelkrämpfe aus. Leichter Mangel kann u. a. im Leistungssport auftreten.

Bislang ist bekannt, wie die einzelnen genannten Substanzen für sich wirken. Bisher ist jedoch noch nicht bekannt, dass die Kombination der genannten Substanzen die häufigsten Beschwerden des Jetlags wie Müdigkeit, Schwindelgefühl, Stimmungsschwankungen, Appetitlosigkeit, verminderte Leistungsfähigkeit bei körperlichen, manuellen und kognitiven Anforderungen und Schlafstörungen schnell beseitigt.

Vitamin B2, Vitamin B12, Vitamin B3, Magnesium, Koenzym Q10, Tryptophan sind notwendige Stoffe, die den Mitochondrienstoffwechsel aktivieren. Z.B. Gibt es in der Atmungskette 3 von 5 Reaktionen, an denen Q10 beteiligt ist.
Ohne Magnesium kann ATP nicht gebildet werden. Carnitin ist an der Mitochondrienmembran zur Stabilisierung notwendig und Fette können nur mit Hilfe von Carnitin in das Mitochondrium eingeschleust werden, deshalb hierdurch verbesserter Energiestoffwechsel.

Galaktose wird in den Stoffwechsel als sofort wirksames Kohlehydrat eingeschleust, Insulin unabhängig.
Vitamin C wirkt auf allen Ebenen auch als Antioxidans.
Da das Gehirn wenige Schutzfaktoren hat, wird es vor oxidativen Prozessen durch Q10, Vitamin C geschützt.

Vorzugsweise umfasst der externe Licht-Dunkel-Wechsel: Tag-Nacht-Rhythmus, Zeitverschiebungen, insbesondere nach Langstreckenflügen, vorzugsweise Jetlag, soziale Zeitgeber, insbesondere erzwungene Veränderungen der Aktivitätsrhythmus bei Schichtarbeit.

Die Komposition eignet sich gut zur Herstellung eines Nahrungsergänzungsmittels, sowohl in fester als auch flüssiger oder Pulverform.

Das entsprechende Nahrungsergänzungsmittel enthaltend die erfindungsgemäße Kombination eignet sich zur Einnahme zur Behandlung einer Störung des circadianen Rhythmus.

In einer besonders bevorzugten Ausführungsform liegen die Einzelkomponenten in der Komposition in nachfolgenden Mol-Verhältnissen zueinander vor:

| Komponente | Verhältnis |
|---|---|
| NADH | 91 - 121 |
| Vitamin B2 | 537 - 716 |
| Vitamin B3 | 125 - 166 |
| Vitamin B12 | 1 |
| Vitamin C | 1912 - 3059 |
| Galaktose | 22450 - 29933 |
| Magnesiumcitrat | 220 - 439 |
| Tryptophan | 990 - 2641 |
| Carnitin | 2510 - 5020 |
| Q10 aktiviert | 78 - 125 |

### Eine besonders bevorzugte Ausführungsform der erfindungsgemäßen Komposition umfasst folgende Mengenbereiche der einzelnen Komponenten:

| | |
|---|---|
| NADH | 30 - 90 mg |
| Vitamin B2 | 100 - 300 mg |
| Vitamin B3 | 50 - 150 mg (als Inositolnicotinat - wirkt retardiert) |
| Vitamin B12 | 500 - 2000 µg (als Hydroxycobalamin) |
| Vitamin C | 200 - 500 mg |
| Galaktose | 2 - 6 g |
| Magnesiumcitrat | 100 - 200 mg |
| Tryptophan | 200 - 500 mg |
| Carnitin | 300 - 600 mg |
| Q10 aktiviert | 40 - 100 mg |

### Als besonders bevorzugte Dosierung hat sich zur Verminderung der Symptome des Jetlags die Gabe von den nachfolgenden Substanzen mit folgender Konzentration herausgestellt:

| | |
|---|---|
| NADH | 30 mg |
| Vitamin B2 | 200 mg |
| Vitamin B3 | 100 mg (als Inositolnicotinat - wirkt retardiert) |
| Vitamin B12 | 1000 µg (als Hydroxycobalamin) |
| Vitamin C | 250 mg |
| Galaktose | 4g |
| Magnesiumcitrat | 150 mg |
| Tryptophan | 250 mg |
| Carnitin | 500 mg |
| Q10 aktiviert | 40 mg |

Ganz besonders bevorzugt werden diese Komponenten und Mengen in mindestens 30 ml Flüssigkeit, insbesondere Wasser, ganz besonders bevorzugt 60 ml gelöst.

Die Wirksamkeit der oben genannten Kombination wurde im Rahmen der erfindungsgemäßen Verwendung zur Erleichterung der Synchronisation eines circadianen Rhythmus durch einen externen Zeitgeber an freiwilligen Probanden nachgewiesen.

Die erfindungsgemäße Verwendung wurde an freiwilligen menschlichen Probanden bei Langstreckenflügen von der Bundesrepublik Deutschland nach USA und China getestet.

Die Dosierung erfolgte unter biochemischen Gesichtspunkten und in Anlehnung an die Empfehlung der handelsüblichen Präparate.

Es wurde subjektiv berichtet, dass sämtliche Probanden die Zeitverschiebung von - 9 Stunden (Los Angeles, Las Vegas) bis + 7 Stunden (Hongkong) innerhalb von ein bis zwei Tagen erreichten, was sich einerseits durch an die neue Zeit angepasste Schlafrhythmen äußerte, andererseits durch eine deutliche Verbesserung der Leistungsfähigkeit bei körperlichen, (manuellen) und kognitiven Anforderungen, schnellem Nachlassen der Müdigkeit und Appetitlosigkeit bereits etwa eine halbe bis eine Stunde nach Einnahme der erfindungsgemäßen Präparate-Kombination.

Sämtliche Probanden unterzogen sich einer Zeitverschiebung durch Langstreckenflug von Frankfurt nach Los Angeles (- 9 Stunden) und zehn weitere Probanden, die sich einer Zeitverschiebung von - 7 Stunden (Langstreckenflug von Frankfurt nach Hongkong) unterzogen, nahmen nach der Ankunft am Zielort sowie am nächsten Morgen nach dem Frühstück jeweils die erfindungsgemäße Kombination und Dosierung der vorgenannten Präparate ein.

Sämtliche Probanden hatten einen mindestens fünftägigen Aufenthalt. Nach Rückkehr nahmen sie direkt nach der Ankunft und auch nach dem Frühstück des darauffolgenden Tages wieder jeweils die erfindungsgemäße Kombination der Präparate ein.

Sie berichteten übereinstimmend und unabhängig voneinander über eine störungsfreie Anpassung an die neue Zeit innerhalb von ein bis zwei Tagen sowie über eine deutliche Besserung der Leistungsfähigkeit bei körperliche, manuellen und kommunikativen Anforderungen, ein schnelles Nachlassen der Müdigkeit und eine Normalisierung des Appetits bereits eine halbe bis eine Stunde nach Einnahme der erfindungsgemäßen Präparate-Kombination sowohl nach der Ankunft als auch nach der Rückkehr.

Zur Überprüfung der Wirksamkeit der Kombination aus NADH und Galaktose wurde, analog dem obigen Vorgehen, die erfindungsgemäße Verwendung zur Erleichterung der Synchronisation eines circadianen Rhythmus durch einen externen Zeitgeber an freiwilligen Probanden nachgewiesen.

Die Verwendung einer Kombination aus 40 mg NADH und 4g Galaktose (zusätzlich mit Aromazusätzen für den Geschmack) wurde dazu wiederum an 23 freiwilligen menschlichen Probanden bei Langstreckenflügen von der Bundesrepublik Deutschland nach USA und Asien getestet.

Sämtliche Probanden klagten über Jetlag-Befindlichkeitsstörungen. Dazu wurden die Schlafstörungen, verstärkte Müdigkeit, Schwindelgefühle, Stimmungsschwankungen, Appetitlosigkeit, verminderte geistige und körperliche Leistungsfähigkeit auf einer Skala von 1 (sehr schwach bis gar nicht) bis 6 (sehr stark) subjektiv bewertet. Die Einstufung der Störungen schwankte zwischen 3 und 6 der Skala. Lediglich die Appetitlosigkeit schwankte zwischen 1 und 3 der Skala.

Sämtliche Probanden nahmen nach der Ankunft am Zielort jeweils einmalig die angegebene Kombination aus NADH und Galaktose ein.

Sie berichteten übereinstimmend und unabhängig voneinander über eine Verbesserung der Befindlichkeitsstörungen um 1 bis 2 Punkte der Skala.

## Patentansprüche

1. Komposition umfassend NADH und D-Galaktose zur Verwendung zur Behandlung einer Störung des circadianen Rhythmus.

2. Komposition zur Verwendung nach Anspruch 1, ferner umfassend Vitamine und/oder Koenzyme und/oder Mineralien

3. Komposition zur Verwendung nach Anspruch 1 oder 2, ferner umfassend mindestens eine oder mehrere Substanzen aus der Gruppe bestehend aus L-Tryptophan, Nikotinsäure oder Nikotinamid, Vitamin B12, Vitamin B2, L-Carnitin, Magnesium, Koenzym Q10, Vitamin C oder Vorläufer der zuvor genannten.

4. Komposition zur Verwendung nach Anspruch 3, **dadurch gekennzeichnet, dass** die Komposition 30 mg NADH, 200 mg Vitamin B2, 100 mg Vitamin B3 als Inositolnicotinat, 1000 µg Vitamin B12 als Hydroxycobalamin, 250 mg Vitamin C, 4 g Galaktose, 150 mg Magnesium als Zitrat, 500mg Tryptophan, 500 mg Carnitin, 100 mg Koenzym Q10 (aktiviert) umfasst.

5. Nahrungsergänzungsmittel enthaltend eine Kombination nach einem der vorhergehenden Ansprüche zur Verwendung zur Behandlung einer Störung des circadianen Rhythmus.

## Claims

1. Composition comprising NADH and D-galactose for use in treating a circadian rhythm disorder.

2. Composition for use as claimed in claim 1, further comprising vitamins and/or coenzymes and/or minerals.

3. Composition for use as claimed in claim 1 or 2, further comprising at least one or more substances from the group consisting of L-tryptophan, nicotinic acid or nicotinamide, vitamin B12, vitamin B2, L-carnitine, magnesium, coenzyme Q10, vitamin C, or precursors thereof.

4. Composition for use as claimed in claim 3, **characterised in that** the composition comprises 30 mg NADH, 200 mg vitamin B2, 100 mg vitamin B3 as inositol nicotinate, 1000 µg vitamin B12 as hydroxycobalamin, 250 mg vitamin C, 4 g galactose, 150 mg magnesium as citrate, 500 mg tryptophan, 500 mg carnitine, and 100 mg coenzyme Q10 (activated).

5. Food supplement containing a combination as claimed in any one of the preceding claims for use in treating a circadian rhythm disorder.

## Revendications

1. Composition comprenant du NADH et du D-galactose, destinée à être utilisée pour le traitement d'un trouble du rythme circadien.

2. Composition destinée à être utilisée selon la revendication 1, comprenant en outre des vitamines et/ou des co-enzymes et/ou des minéraux.

3. Composition destinée à être utilisée selon la revendication 1 ou 2, comprenant en outre au moins une ou plusieurs substances dans le groupe constitué par le L-tryptophane, l'acide nicotinique ou le nicotinamide, la vitamine B12, la vitamine B2, la L-carnitine, le magnésium, la coenzyme Q10, la vitamine C ou un précurseur de ceux-ci.

4. Composition destinée à être utilisée selon la revendication 3, **caractérisée en ce que** la composition contient 30 mg de NADH, 200 mg de vitamine B2, 100 mg de vitamine B3 sous la forme d'inositol, 1000 µg de vitamine B12 sous la forme d'hydroxycobalamine, 250 mg de vitamine C, 4 g de galactose, 150 mg de magnésium sous la forme de citrate, 500 mg de tryptophane, 500 mg de carnitine, 100 mg de coenzyme Q10 (activée).

5. Compléments alimentaires contenant une combinaison selon l'une quelconque des revendications précédentes, destinés à être utilisés dans le traitement d'un trouble du rythme circadien.
